# EUROPEAN PATENT SPECIFICATION

(11) **EP 0 357 550 B1**
(45) Date of publication and mention of the grant of the patent: **02.03.1994**
(21) Application number: 89810565.5
(22) Date of filing: 25.07.1989
(51) Int. Cl.: A61K 31/47

(54) **New use of 5-heteroaryl- or 5-aryl-substituted imidazo [2,1-a] isoquinolines**
Verwendung von 5-heteroaryl- oder 5-arylsubstituierten Imidazo(2,1-a)isochinolinen
Utilisation d'imidazo(2,1-a)isoquinolines substituées sur la position 5 par des groupes hétéroaryles ou aryles

(30) Priority: 29.07.1988 US 226305; 27.01.1989 US 302720
(43) Date of publication of application: 07.03.1990
(73) Proprietor: SANDOZ AG, 4002 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Houlihan, William Joseph, Mountain Lakes, N.J. 07046 (US)

(56) References cited:
- WO-A-88/00587
- JOURNAL OF PHARMACOLOGY AND EXPERIMENTAL THERAPEUTICS, vol. 247, no. 2, November 1988, pages 617 - 623; D.A. HANDLEY et al.: "Biological Effects of the Orally Active Platelet Activating Factor Receptor Antagonist SDZ 64-412"

## Description

The present invention relates to a novel use of certain 5-heteroaryl- or 5-aryl-substituted imidazo[2,1-a]isoquinolines in the prophylactic treatment of asthma.

In particular, the invention concerns the new use of the compounds of formula I 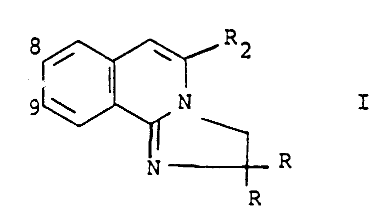
wherein each R independently is hydrogen or methyl,
and
R₂ represents a group 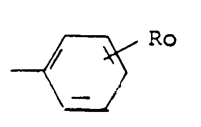
wherein Ro represents
a group 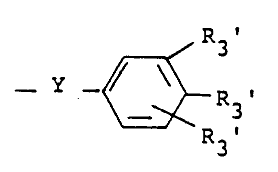
wherein each R₃' represents independently C₁₋₃alkoxy and
Y represents -O(CH₂)₁₋₅-, -(CH₂)₁₋₆-or -CH₂OCH₂- ; with the proviso that Ro may only be in a meta or para position
and their pharmaceutically acceptable acid addition salts where such may exist in the preparation of a pharmaceutical composition for use in the prophylactic treatment of asthma.

Alkyl moieties may be straight chained or branched.

A particular compound group covers those of formula I wherein
R₂ is a group 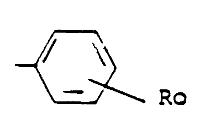
wherein Ro represents
a group 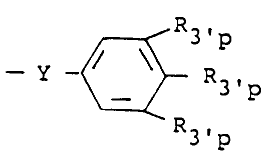
wherein each R₃,ₚ represents C₁₋₃alkoxy and
Y represents -OCH₂, -CH₂-, -CH₂CH₂- or
CH₂OCH₂, and their pharmaceutically acceptable acid addition salts where such may exist.

In another compound group of formula I
R₂ represents a group 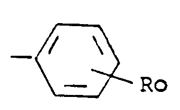
wherein Ro represents
a group 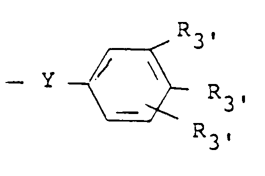
wherein each R₃, represents independently C₁₋₃alkoxy and
Y represents -OCH₂-, -CH₂OCH₂-, -CH₂-, -CH₂CH₂- or -CH₂CH₂CH₂-with the proviso that Ro may only be in a meta- or para-position.

Preferred is especially 5-[4'-(3,4,5-trimethoxyphenylethyl) phenyl]-2,3-dihydroimidazo-[2,1-a]isoquinoline, especially the hydrochloride thereof, i.e. compound 7E) hereunder.

Especially preferred pharmaceutically acceptable acid addition salts are the hydrochloride acid addition salts.

The compounds for use in the invention are known and may be prepared e.g. as described in WO 88/06157.

As is evident to those skilled in the art, the compounds of formula I may exist in racemic or enantiomeric form and the invention covers all forms. Enantiomeric forms may be recovered in conventional manner e.g. by resolution of end or intermediate products or by employing optically active starting materials.

Examples of pharmaceutically acceptable acid addition salts include those with mineral acids, e.g. hydrochloric, hydrobromic, phosphoric and sulfuric acid and organic acids, e.g. tartaric, acetic, citric, malic, maleic, methanesulphonic and gluconic acid which may be prepared in conventional manner.

A relationship between PAF antagonism and certain pulmonary conditions is known in the art. Thus, e.g. WO 88/06157 discloses that the compounds of formula I are indicated for use e.g. in PAF-mediated bronchoconstriction or in PAF-mediated, endotoxin-induced lung injury and, analogously, endotoxin-induced septic shock and adult respiratory distress syndrome. These conditions are acute manifestations of disease and, as is well-known, allow no prediction as to the usefulness of drugs effective in treating them, in the prophylactic treatment of further conditions closely or distantly related therewith.

It has now been found that, surprisingly, the compounds of formula I are indicated for use in the prophylactic treatment of asthma.

This property can be measured upon oral administration in accordance with the following procedure (test A) :
Male Hartley guinea pigs weighing 250 g are sensitized to ovalbumin by aerosol inhalation exposure. The test animals are then subsequently rechallenged with ovalbumin aerosol repeatedly (3 to 6 times) over a period of two to three weeks. Airway reactivity is assessed by an acetylcholine dose response curve at times (1 to 3 days) after the last ovalbumin exposure. The test compound is assessed for its ability to control hyperreactive airways by administering it orally with a gavage tube in an acceptable vehicle prior to each ovalbumin allergen exposure.

The following result is obtained with compound 7E) when administered 2 hours before the antigen challenge: ED₅₀ = 10 mg/kg; whereas prednisone at 5 mg/kg did not inhibit the development of airways reactivity under the same conditions.

The effect on airways hyperreactivity may also be determined by the effect of a test compound upon the pulmonary eosinophilia that follows exposure to PAF (test AA) . The test method is as follows:
The test compounds are dissolved in saline (0.9 %); dexamethasone (Sigma) is dissolved in ethanol (50 % v/v) : polyethylene glycol (50 % v/v). The test compound is loaded into osmotic minipumps (Alzet) and implanted subcutaneously in the nuchal region of male Dunken-Hartley guinea pigs (350-450 g) under ether anaesthesia (the compound 7E) was administered per os 1 hour before exposure to PAF). Five days after implantation of minipumps, animals are exposed for one hour to an aerosol of PAF (100 µg in 10 ml 0.25 % w/v bovine serum albumin) in four litre chambers supplied with air at 7 litres per minute via a Devillbiss nebuliser. Forty-eight hours later animals are killed by an overdose of barbiturate (200 mg/kg ip) Tracheae are cannulated and airways are lavaged with sic 10 ml volumes of modified Tyrode solution containing EDTA (19.8 mM), gelatin (0.1 % w/v) and BSA (0.5 % w/v). Total cell numbers recovered by broncho-alveolar lavage (BAL) are enumerated using a haemocytometer, and differential counts of 500 cells made from smears stained with Leishman's stain.

The effect of test compounds on eosinophil accumulation is summarized in Table 1. The total number of cells recovered by BAL is not increased following exposure to PAF. Exposure to PAF causes an increase in the percentage of eosinophils recovered in BAL from 9.8 ± 1.6 % (mean ± standard error of the mean, BSA aerosol control) to 22.2 ± 2.0 %. Pretreatment of guinea pigs with cromoglycate or compound 7E) causes a significant decrease in the prevalence of eosinophils in BAL.

**Table 1**

| Effect on PAF-induced eosinophil accumulation in pulmonary airways of the guinea pig | | | | | |
|---|---|---|---|---|---|
| Test compound | Dose (mg/kg/d) | n | Total white cell count in BAL (x10⁶) | Eosinophils (5) | Probability* versus saline |
| saline° | - | 10 | 20.0 ± 2.1 | 22.2 ± 1.6 | |
| Cromoglycate | 1.0 | 10 | 17.8 ± 2.3 | 13.4 ± 2.0 | < 0.01 |
| Compound 7E) | 10.0 | 10 | 24.7 ± 4.4 | 10.0 ± 2.5 | < 0.001 |

| | | | | | |
|---|---|---|---|---|---|
| * Probability by Student's t test | | | | | |
| ° Total leukocyte count in control animals (n = 10) not exposed to PAF aerosol was 20.8 ± 2.4 x 10, of which 5.0 ± 0.7 % were eosinophils | | | | | |

It can be seen that Cromoglycate and compound 7E) both share the capacity to inhibit the induction of airways hyperreactivity by PAF as evidenced by the capacity to inhibit eosinophil accumulation. That capacity may be viewed as a prerequisite for prophylactic efficacy in the treatment of asthma.

This effect is further confirmed as follows (test AB) :
PAF-induced cellular recruitment in guinea pig airways is initiated by bronchoalveolar lavage (BAL) and the activity of the test compound is measured by its ability to inhibit the response. Animals (n = 3 per group, repeated twice) are exposed for 5 minutes to nebulized PAF (C₁₈: 1-1000 µg/ml), lyso-PAF or controls (0.25 % guinea pig albumin-saline). BAL is done 1 to 96 hours post challenge using 5 x 10 ml of a modified Tyrode's solution. Values are mean±sem of cell counts (x 10³/mm³) using a Sysmex blood analyzer for total leukocytes and a manual differential count. A Student's t-test is used for analysis. PAF at 1000 µg/ml results in a maximal 12 x increase (6.29 ± 1.39) at 72 hours over lyso-PAF or vehicle (0.49 ± 0.18). Test compounds are given at 24 and 6 hours pre-PAF and 1 x 3 days.

The results obtained are summarized in Table 2:

**Table 2**

| Compound | Dosage (mg/kg po) | Eosinophils |
|---|---|---|
| Compound 7E) | 20 | 3.4 ± 0.8 (p<0.01) |
| | 40 | 1.9 ± 0.7 (p<0.01) |

It can be seen that compound 7E) again elicits marked inhibition of eosinophilia in the guinea pig airways typical of prophylactic asthma drugs.

The inhibition of airways hyperreactivity can also be assessed when the test compound is administered topically, i.e. by the inhalation route, according to the following procedure (test B) :

Male Hartley guinea pigs weighing 250 g are sensitized to ovalbumin (OA) by a series of three peritoneal injections of OA over a period of six weeks. On the day of the study, the test compound is topically (i.e., by inhalation) administered to the test animals by aerosol exposure prior to allergen exposure. The test animals are restrained in a small plexiglas chamber to allow head-only exposure to aerosol administration of the test compound. Aerosol is generated by an ultrasonic nebulizer and the output is directed into the head chamber of the test animal box. The test animals are exposed in the manner for 20 minutes immediately preceding allergen exposure. Allergen exposure consists of 60 minutes of aerosol OA exposure. Airways reactivity is assessed by an allergen dose response curbe at 24 hours after allergen exposure.

Employing the above procedure, the ability of the compound 7E) to control hyperreactive airways was demonstrated by a histamine dose response curve in the following groups of test animals:
- Group 1 -: Control test animals which were neither exposed to the test compound nor to allergen. This group represents baseline airway reactivity;
- Group 2 -: Allergen-only test animals which were not exposed to the test compound but were exposed to aerosol allergen. This group represents the no-drug effect;
- Group 3 -: Test animals which were treated by aerosol exposure to a 2 % solution of the test compound in water for 70 minutes prior to allergen exposure;
- Group 4 -: Test animals which were treated by aerosol exposure to a 1 % solution of the test compound in water for 20 minutes prior to allergen exposure;
- Group 5 -: Test animals which were treated by aerosol exposure to a 0.1 % solution of the test compound in water for 20 minutes prior to allergen exposure.

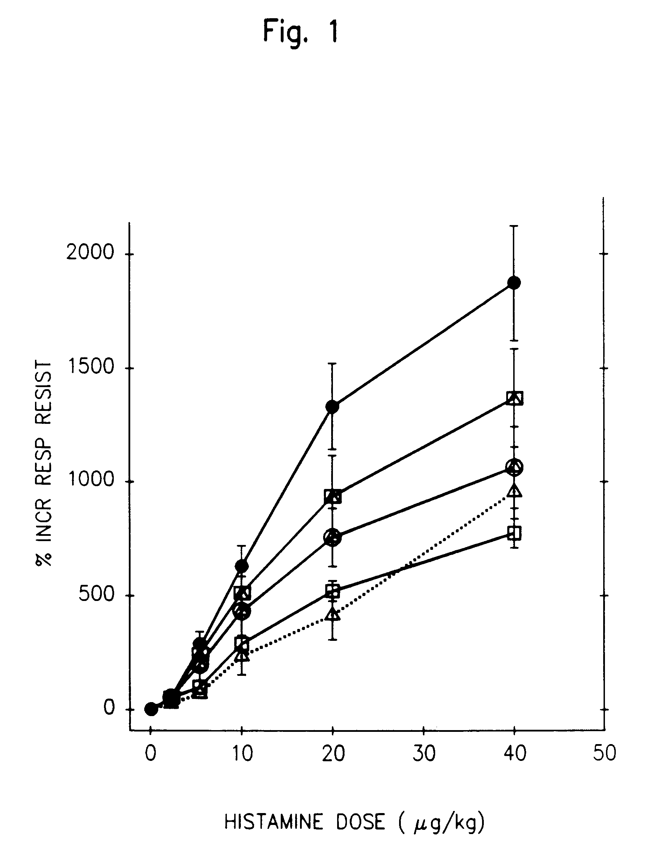

As can be seen from the results obtained, which are set forth in Figure 1, Compound 7E) is highly effective in controlling allergen-induced hyperreactive airways by the inhalation route.

The compound of formula I and their pharmaceutically acceptable acid addition salts may be administered by any conventional route for use in the indication disclosed with the present invention.

However, it has been surprisingly found that when a compound of formula I or a pharmaceutically acceptable acid addition salt thereof is administered by the inhalation route, the results are superior to those obtained by enteral, e.g. oral administration. The inhalation mode of administration is particularly suitable because it allows for higher local tissue drug concentration in the organ of interest, e.g. the lung, and results in a much lower total delivered dose, thereby drastically reducing the possibility of systemic toxicity or side effects. Such type compositions may be in conventional form for systemic administration of the active agent through the pulmonary mucous membrane employing conventional pulmonary applicators which may be e.g. an aerosol for inhalation or a powder spray device for inhalation. Inhalation may e.g. be by the oral or nasal route.

The compounds are thus indicated for use in the prophylactic treatment of asthma. An indicated suitable daily dosage for this use when administered orally is from about 10 mg to about 2000 mg, preferably from about 10 mg to about 350 mg, suitably administered in divided dosages of from about 0.25 mg to about 500 mg, especially from about 0.25 mg to about 350 mg, up to four times daily or in controlled release form. A typical oral dosage is 50 mg or 100 mg two or three times a day. A typical oral unit dosage may contain from about 2.5 mg to about 500 mg, preferably from about 5 mg to about 200 mg, especially from about 10 mg to about 100 mg of active substance.

An indicated suitable daily dosage for this use when administered by the inhalation route is a dose which is between about 10 and about 1000 times lower than the oral dose. Satisfactory daily doses for the use by the inhalation route may be determined from the dosage regimen indicated above regarding oral administration.

The invention thus concerns the use of the compounds of formula I and their pharmaceutically acceptable acid addition salts where such may exist, in the prophylactic treatment of asthma.

It also concerns pharmaceutical compositions for use in the above indication.

It also concerns pharmaceutical compositions suitable for administration by the inhalation route containing a compound of formula I or a pharmaceutically acceptable acid addition salt thereof where such may exist, e.g. as part of a nebulizer or a powder spray device.

Pharmaceutical compositions suitable for administration by the inhalation route are known in the art. The test compound is dissolved in a suitable vehicle or employed as a fine powder in a 5-10 micron particle size and placed in a device capable of delivering metered doses of the test compound by e.g. the oral inhalation route. A propellant such as dichlorotetrafluoroethane (DCTFE) and/or dichlorodifluoroethane (DCDFE) is used as the pressurizing agent. A typical formulation for use in oral inhalation contains, by volume, between 5 and 15 % of the test compound, between 82 and 84 % of propellant such as DCTFE and/or DCDFE and between 1 and 3 % of a wetting agent such as trioleate.

The following compounds of formula I are particularly suitable for the above new use:
3 HH) 5-[4'-(3,4,5-trimethoxybenzyloxy)phenyl]-2,3-dihydroimidazo[2,1-a]isoquinoline hydcochloride
3 OO) 5-[4'-(3,4,5-trimethoxyphenylpropyloxy)phenyl]-2,3-di hydroimidazo[2,1-a]isoquinoline hydrochloride
3 PP) 5-[4'-(3,4,5-trimethoxyphenylhexyl)phenyl]-2,3-dihydroimidazo[2,1-a]isoquinoline hydrochloride
3 QQ) 5-[4'-(3,4,5-trimethoxyphenylethyloxy)phenyl]-2,3-dihydroimidazo[2,1-a]isoquinoline hydrochloride
5 E) 2,2-dimethyl-5-[4'-(3,4,5-trimethoxyphenylethyl)phenyl]-2,3-dihydroimidazo[2,1-a]isoquinoline hydrochloride
7 A) 5-[4'-(3,4,5-trimethoxybenzyloxymethyl)phenyl]-2,3-dihydroimidazo[2,1-a]isoquinoline hydrochloride
7 C) 5-[3'-(3,4,5-trimethoxybenzyloxy)phenyl]-2, 3-dihydroimidazo[2,1-a]isoquinoline hydrochloride,
7 D) 5-[2'-(3,4,5-trimethoxybenzyloxy)phenyl]-2, 3-dihydroimidazo[2,1-a]isoquinoline hydrochloride,
7 E) 5-[4'-(3,4,5-trimethoxyphenylethyl)phenyl]-2, 3-dihydroimidazo [2,1-a]isoquinoline hydrochloride,
7 F) 5-[3'-(3^{,}4,5-trimethoxyphenylethyl)pnenyl]-2, 3-dihydroimidazo[2,1-a]isoquinoline hydrochloride,
7 K) 5-[4'-(3,4^{,}5-trimethoxyphenylpropyl)pnenyl]-2, 3-dihydroimidazo[2,1-a]isoquinoline hydrochloride,
7 L) 5-[4'-(2,3,4-trimethoxybenzyloxy)pnenyl]-2, 3-dihydroimidazo[2,1-a]isoquinoline hydrochloride, and
7 N) 5-[4'-(3,4,5-trimethoxybenzyl)phenyl]-2, 3-dihydroimidazo[2,1-a]isoquinoline hydrochloride.

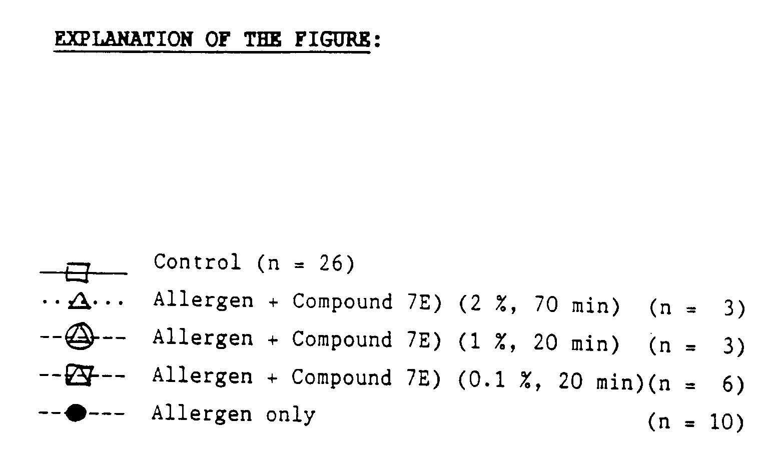

## Claims (Claims for the following Contracting State(s): AT, BE, CH, DE, FR, GB, IT, LI, LU, NL, SE)

1. The use of the compounds of formula I 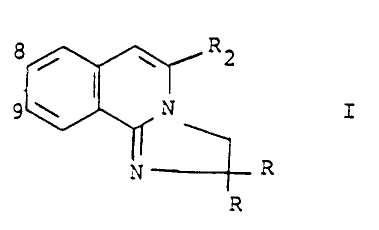 wherein each R independently is hydrogen or methyl, and
R₂ represents a group 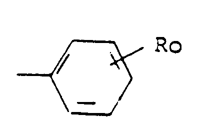 wherein Ro represents
a group 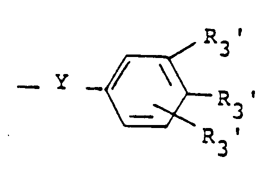 wherein each R₃' represents independently C₁₋₃alkoxy and
Y represents -O(CH₂)₁₋₅-, -(CH₂)₁₋₆-or -CH₂OCH₂- ; with the proviso that Ro may only be in a meta or para position
and their pharmaceutically acceptable acid addition salts where such may exist in the preparation of a pharmaceutical composition for use in the prophylactic treatment of asthma.

2. The use according to claim 1 of the compound 5-[4'-(3,4,5-trimethoxyphenylethyl)phenyl]2,3-dihydroimidazo[2,1-a]isoquinoline and its pharmaceutically acceptable acid addition salts.

3. The use according to claim 1 of the compound 5-[4'-(3,4,5-trimethoxyphenylethyl)phenyl]-2,3-dihydroimidazo[2,1-a]isoquinoline hydrochloride.

4. A pharmaceutical composition comprising a compound of formula I as defined in claim 1 or a pharmaceutically acceptable acid addition salt thereof where such may exist as active ingredient together with one or more pharmaceutically acceptable carrier or diluent therefor and suitable or adapted for administration only by the inhalation route to the exclusion of oral and injectable administration.

5. A pharmaceutical composition according to claim 4 comprising a compound as defined in claim 2.

6. A pharmaceutical composition according to claim 4 comprising a compound as defined in claim 3.

7. A pharmaceutical composition according to claim 4 which is part of a nebulizer or powder spray device.

8. A pharmaceutical composition according to claim 4 for use in oral inhalation comprising by volume between 5 and 15 % of the compound, between 82 and 84 % of a propellant and between 1 and 3 % of a wetting agent.

## Claims (Claims for the following Contracting State(s): ES, GR)

1. The use of the compounds of formula I 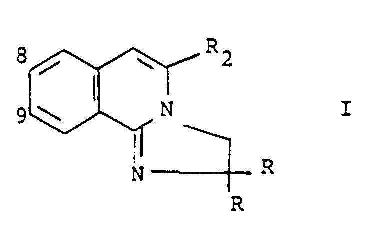 wherein each R independently is hydrogen or methyl, and
R₂ represents a group 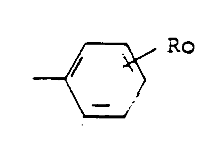 wherein Ro represents
a group 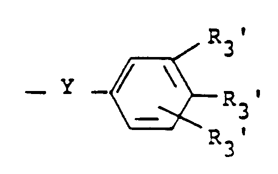 wherein each R₃' represents independently C₁₋₃alkoxy and
Y represents -O(CH₂)₁₋₅-, -(CH₂)₁₋₆-
or -CH₂OCH₂- ; with the proviso that Ro may only be in a meta or para position
and their pharmaceutically acceptable acid addition salts where such may exist in the preparation of a pharmaceutical composition for use in the prophylactic treatment of asthma.

2. The use according to claim 1 of the compound 5-[4'-(3,4,5-trimethoxyphenylethyl)phenyl]2,3-dihydroimidazo[2,1-a]isoquinoline and its pharmaceutically acceptable acid addition salts.

3. The use according to claim 1 of the compound 5-[4'-(3,4,5-trimethoxyphenylethyl)phenyl]-2,3-dihydroimidazo[2,1-a]isoquinoline hydrochloride.

4. A process for the preparation of a pharmaceutical composition for use in the prophylactic treatment of asthma comprising mixing a compound of formula I as defined in claim 1 or a pharmaceutically acceptable acid addition salt thereof where such may exist, with a pharmaceutically acceptable carrier or diluent, said composition being suitable or adapted for administration only by the inhalation route to the exclusion of oral and injectable administration.

5. A process according to claim 1 comprising mixing a compound as defined in claim 2.

6. A process according to claim 1 comprising mixing a compound as defined in claim 3.

## Patentansprüche (Patentansprüche für folgende(n) Vertragsstaat(en): AT, BE, CH, DE, FR, GB, IT, LI, LU, NL, SE,)

1. Verwendung der Verbindungen der Formel I 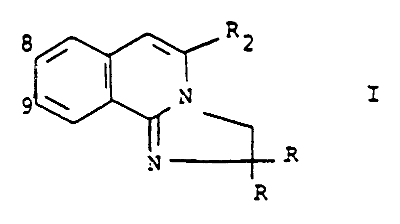 worin
jeder Rest R unabhängig Wasserstoff oder ein Methylrest ist, R₂ eine Gruppe 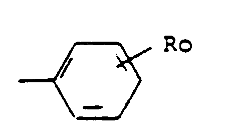 bedeutet, worin Ro eine Gruppe 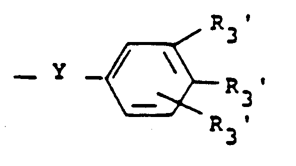 bedeutet, worin jeder Rest R₃' unabhängig eine C₁-C₃-Alkoxygruppe bedeutet und Y -O(CH₂)₁₋₅-, -(CH₂)₁₋₆- oder -CH₂OCH₂-bedeutet; mit dem Vorbehalt, daß Ro nur in meta- oder para-Stellung sein kann;
und deren pharmazeutisch annehmbare Säureadditionssalze, falls diese existieren, zur Herstellung einer pharmazeutischen Zusammensetzung zur Verwendung zur prophylaktischen Behandlung von Asthma.

2. Verwendung nach Anspruch 1 der Verbindung 5-[4'-(3,4,5-Trimethoxyphenylethyl)-phenyl]-2,3-dihydroimidazo[2,1-a]isochinolin und deren pharmazeutisch annehmbaren Säureadditionssalzen.

3. Verwendung nach Anspruch 1 der Verbindung 5-[4'-(3,4,5-Trimethoxyphenylethyl)-phenyl]-2,3-dihydroimidazo[2,1-a]isochinolin-hydrochlorid.

4. Pharmazeutische Zusammensetzung umfassend eine Verbindung der Formel I, wie in Anspruch 1 definiert, oder ein pharmazeutisch annehmbares Säureadditionssalz davon, falls dieses existiert, als aktiver Inhaltsstoff zusammen mit einem oder mehreren pharmazeutisch annehmbaren Trägern oder Verdünnungsmitteln dafür, die geeignet oder angepaßt ist zur Verabreichung auf dem Inhalationsweg unter Ausschluß der oralen und injizierbaren Verabreichung.

5. Pharmazeutische Zusammensetzung nach Anspruch 4, umfassend eine Verbindung wie in Anspruch 2 definiert.

6. Pharmazeutische Zusammensetzung nach Anspruch 4, umfassend eine Verbindung wie in Anspruch 3 definiert.

7. Pharmazeutische Zusammensetzung nach Anspruch 4, die Teil eines Verneblers oder einer Pulversprühvorrichtung ist.

8. Pharmazeutische Zusammensetzung nach Anspruch 4 zur Verwendung für die orale Inhalation, umfassend, bezogen auf Volumen, 5 bis 15% der Verbindung, 82 bis 84% eines Treibmittels und 1 bis 3% eines Benetzungsmittels.

## Patentansprüche (Patentansprüche für folgende(n) Vertragsstaat(en): ES, GR)

1. Verwendung der Verbindungen der Formel I 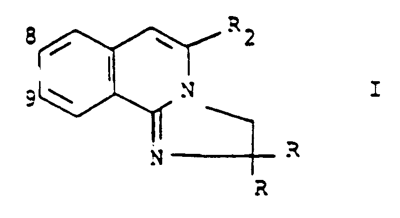 worin
jeder Rest R unabhängig Wasserstoff oder ein Methylrest ist, R₂ eine Gruppe 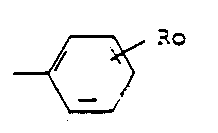 bedeutet, worin Ro eine Gruppe 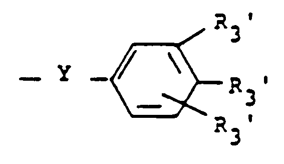 bedeutet, worin jeder Rest R₃' unabhängig eine C₁-C₃-Alkoxygruppe bedeutet und Y -O(CH₂)₁₋₅-, -(CH₂)₁₋₆- oder -CH₂OCH₂-bedeutet; mit dem Vorbehalt, daß Ro nur in meta- oder para-Stellung sein kann;
und deren pharmazeutisch annehmbare Säureadditionssalze, falls diese existieren, zur Herstellung einer pharmazeutischen Zusammensetzung zur Verwendung zur prophylaktischen Behandlung von Asthma.

2. Verwendung nach Anspruch 1 der Verbindung 5-[4'-(3,4,5-Trimethoxyphenylethyl)-phenyl]-2,3-dihydroimidazo[2,1-a]isochinolin und deren pharmazeutisch annehmbaren Säureadditionssalzen.

3. Verwendung nach Anspruch 1 der Verbindung 5-[4'-(3,4,5-Trimethoxyphenylethyl)-phenyl]-2,3-dihydroimidazo[2,1-a]isochinolin-hydrochlorid.

4. Verfahren zur Herstellung einer pharmazeutischen Zusammensetzung zur Verwendung zur prophylaktischen Behandlung von Asthma, umfassend, daß man eine Verbindung der Formel I, wie in Anspruch 1 definiert, oder ein pharmazeutisch annehmbares Säureadditionssalz davon, falls dieses existiert, mit einem pharmazeutisch annehmbaren Träger oder Verdünnungsmittel vermischt, wobei die Zusammensetzung geeignet oder angepaßt ist zur Verabreichung nur auf dem Inhalationsweg unter Ausschluß einer oralen und injizierbaren Verabreichung.

5. Verfahren nach Anspruch 4, umfassend, daß man eine Verbindung, wie in Anspruch 2 definiert, vermischt.

6. Verfahren nach Anspruch 4, umfassend, daß man eine Verbindung, wie in Anspruch 3 definiert, vermischt.

## Revendications (Revendications pour l'(les) Etat(s) contractant(s) suivant(s): AT, BE, CH, DE, FR, GB, IT, LI, LU, NL, SE)

1. L'utilisation des composés de formule I 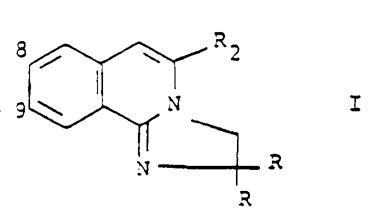 dans laquelle
chaque R signifie indépendamment l'hydrogène ou un groupe méthyle, et
R₂ signifie un groupe 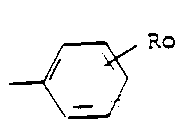 où Ro signifie un groupe 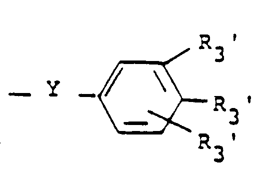 où chaque R₃' signifie indépendamment un groupe alcoxy en C₁-C₃ et
Y signifie un groupe -O(CH₂)₁₋₅-, -(CH₂)₁₋₆- ou -CH₂OCH₂-, avec la condition que Ro puisse seulement être en position méta ou para, et leurs sels d'addition d'acides pharmaceutiquement acceptables lorsque ceux-ci peuvent exister, dans la préparation d'une composition pharmaceutique destinée à l'utilisation dans le traitement prophylactique de l'asthme.

2. L'utilisation selon la revendication 1 de la 5-[4'-(3,4,5-triméthoxyphényléthyl)phényl]2,3-dihydroimidazo[2,1-a]isoquinoléine et de ses sels d'addition d'acides pharmaceutiquement acceptables.

3. L'utilisation selon la revendication 1 du chlorhydrate de 5-[4'-(3,4,5-triméthoxyphényléthyl)phényl]-2,3-dihydroimidazo[2,1-a]isoquinoléine.

4. Une composition pharmaceutique comprenant comme substance active un composé de formule I tel que défini à la revendication 1 ou un sel d'addition d'acide pharmaceutiquement acceptable de ce composé lorsque celui-ci peut exister, ensemble avec un ou plusieurs véhicules ou diluants pharmaceutiquement acceptables, et appropriée ou adaptée pour l'administration uniquement par inhalation, l'administration par voie orale et par injection étant exclues.

5. Une composition pharmaceutique selon la revendication 4, comprenant un composé tel que défini à la revendication 2.

6. Une composition pharmaceutique selon la revendication 4, comprenant un composé tel que défini à la revendication 3.

7. Une composition pharmaceutique selon la revendication 4 qui fait partie d'un nébuliseur ou d'un dispositif de pulvérisation d'une poudre.

8. Une composition pharmaceutique selon la revendication 4, pour l'utilisation pour l'inhalation orale comprenant en volume entre 5 et 15% du composé, entre 82 et 84% d'un propulseur et entre 1 et 3% d'un agent mouillant.

## Revendications (Revendications pour l'(les) Etat(s) contractant(s) suivant(s): ES, GR)

1. L'utilisation des composés de formule I 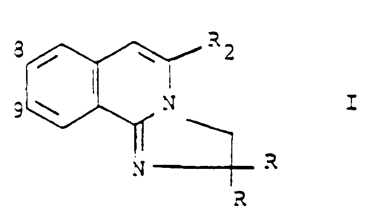 dans laquelle
chaque R signifie indépendamment l'hydrogène ou un groupe méthyle, et
R₂ signifie un groupe 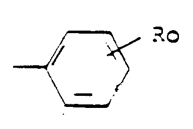 où Ro signifie un groupe 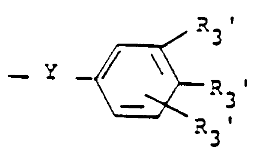 où chaque R₃' signifie indépendamment un groupe alcoxy en C₁-C₃
et
Y signifie un groupe -O(CH₂)₁₋₅-, -(CH₂)₁₋₆- ou -CH₂OCH₂-, avec la condition que Ro puisse seulement être en position méta ou para, et leurs sels d'addition d'acides pharmaceutiquement acceptables lorsque ceux-ci peuvent exister, dans la préparation d'une composition pharmaceutique destinée à l'utilisation dans le traitement prophylactique de l'asthme.

2. L'utilisation selon la revendication 1 de la 5-[4'-(3,4,5-triméthoxyphényléthyl)phényl]-2,3-dihydroimidazo[2,1-a]isoquinoléine et de ses sels d'addition d'acides pharmaceutiquement acceptables.

3. L'utilisation selon la revendication 1 du chlorhydrate de 5-[4'-(3,4,5-triméthoxyphényléthyl)phényl]-2,3-dihydroimidazo[2,1-a]isoquinoléine.

4. Un procédé de préparation d'une composition pharmaceutique pour l'utilisation dans le traitement prophylactique de l'asthme, comprenant le mélange d'un composé de formule I tel que défini à la revendication 1 ou un sel d'addition d'acide pharmaceutiquement acceptable de ce composé lorsque celui-ci peut exister, avec un véhicule ou diluant pharmaceutiquement acceptable, ladite composition étant appropriée ou adaptée pour l'administration uniquement par inhalation, l'administration par voie orale et par injection étant exclues.

5. Un procédé selon la revendication 4, comprenant le mélange d'un composé tel que défini à la revendication 2.

6. Un procédé selon la revendication 4, comprenant le mélange d'un composé tel que défini à la revendication 3.
